# EUROPEAN PATENT APPLICATION

(11) **EP 4 530 977 A1**
(43) Date of publication of application: **02.04.2025**
(21) Application number: 24161113.6
(22) Date of filing: 04.03.2024
(51) Int. Cl.: G06T 7/00, H04N 1/00

(54) **IMAGE QUALITY EVALUATION ACCORDING TO ACTUAL USAGE**

(30) Priority: 26.09.2023 JP 2023163149
(71) Applicant: Fujifilm Business Innovation Corp., Tokyo 107-0052 (JP)
(72) Inventor: HASEGAWA, Maki, Yokohama, 220-8668 (JP); TOMODA, Kyotaro, Yokohama, 220-8668 (JP); TAKAHASHI, Kazuyuki, Yokohama, 220-8668 (JP)
(74) Representative: Meissner Bolte Partnerschaft mbB

(57) **Abstract**

An information processing system includes at least one processor configured to: obtain image data to be diagnosed; evaluate the image data on the basis of evaluation criteria for each of one or more evaluation items related to image quality; and allow an evaluation method based on the evaluation criteria to be changed according to an evaluation result in the evaluation of each of the evaluation items.

## Description

### Background

### (i) Technical Field

The present disclosure relates to an information processing system, a diagnostic system, an information processing method, and a program.

### (ii) Related Art

Japanese Unexamined Patent Application Publication No. 9-218956 discloses a method for predicting the overall color image quality score using, as variables, image quality psychophysical quantities that represent psychological quantities of individual image quality psychological factors that contribute to the overall color image quality score of a recording device or a display device, wherein for each of the image quality psychological factors, the relationship between the image quality psychophysical quantities and the overall color image quality score obtained for existing recording devices or display devices is stored in memory as statistical data in advance, and the overall color image quality score of a recording device or a display device to be evaluated is predicted statistically using the statistical data, namely the relationships between the image quality psychophysical quantities and the overall color image quality score, in the memory.

Japanese Unexamined Patent Application Publication No. 10-63859 discloses the following: learning a relationship between an overall image quality score determined by a subject, features of partial images at positions in an image that the subject focuses on when determining the overall image quality score, and the image quality rating calculated according to such features; identifying, on the basis of the learning result, the positions of partial images to be evaluated in relation to image quality evaluation items required to determine an overall image quality score for an image under evaluation; calculating image quality ratings for the image quality evaluation items required to determine an overall image quality score with respect to the partial image information at the identified positions; and calculating an overall image quality score for the image under evaluation on the basis of the calculated image quality ratings and the learning result.

### Summary

In image diagnosis, image quality is evaluated on the basis of criteria for each of predetermined items. However, it is difficult to establish uniform criteria for image quality evaluation because the degree of acceptable image quality for each evaluation item is not the same depending on the user.

Accordingly, it is an object of the present disclosure to provide a system capable of returning an image quality evaluation according to actual usage by the user, as compared to a configuration that evaluates image quality on the basis of indiscriminate criteria.

According to a first aspect of the present disclosure, there is provided an information processing system including at least one processor configured to:
obtain image data to be diagnosed;
evaluate the image data on the basis of evaluation criteria for each of one or more evaluation items related to image quality; and
allow an evaluation method based on the evaluation criteria to be changed according to an evaluation result in the evaluation of each of the evaluation items.

According to a second aspect of the present disclosure, in the information processing system according to the first aspect, the processor is configured such that when the evaluation result meets a predetermined condition in relation to one evaluation item, the processor allows the evaluation item to be excluded from evaluation.

According to a third aspect of the present disclosure, in the information processing system according to the second aspect, the processor is configured such that when the evaluation result meets a second condition different from the predetermined condition in relation to one evaluation item, the processor allows the evaluation criterion for the evaluation item to be relaxed.

According to a fourth aspect of the present disclosure, in the information processing system according to any one of the first to third aspects, the processor is configured such that when evaluation results from multiple consecutive evaluations meet a predetermined condition in relation to one evaluation item, the processor allows the evaluation method regarding the evaluation item to be changed.

According to a fifth aspect of the present disclosure, in the information processing system according to the fourth aspect, the processor is configured such that when evaluation results from multiple evaluations meet a predetermined condition in relation to one evaluation item in an ongoing way, the processor allows the evaluation method regarding the evaluation item to be changed.

According to a sixth aspect of the present disclosure, there is provided a diagnostic system including:
the information processing system according to any one of the first to fifth aspects;
an image processing device that forms an image on a recording medium; and
a reading device that reads an image formed on a recording medium by the image processing device and creates image data to be diagnosed.

According to a seventh aspect of the present disclosure, there is provided an information processing method including:
obtaining image data to be diagnosed;
evaluating the image data on the basis of evaluation criteria for each of one or more evaluation items related to image quality; and
allowing an evaluation method based on the evaluation criteria to be changed according to an evaluation result in the evaluation of each of the evaluation items.

According to an eighth aspect of the present disclosure, there is provided a program causing a computer to execute a process including:
obtaining image data to be diagnosed;
evaluating the image data on the basis of evaluation criteria for each of one or more evaluation items related to image quality; and
allowing an evaluation method based on the evaluation criteria to be changed according to an evaluation result in the evaluation of each of the evaluation items.

According to the first aspect of the present disclosure, it is possible to return an image quality evaluation that reflects actual usage by the user, as compared to a configuration that evaluates image quality on the basis of indiscriminate criteria.

According to the second aspect of the present disclosure, an evaluation item that the user deems unnecessary may be excluded from evaluation.

According to the third aspect of the present disclosure, the evaluation criterion for an evaluation item that the user does not scrutinize may be relaxed.

According to the fourth aspect of the present disclosure, user preferences with respect to image quality may be reflected, unlike a configuration that makes a determination on the basis of a single evaluation result.

According to the fifth aspect of the present disclosure, it is possible to exclude cases in which the evaluation results vary irregularly due to a fault or the like.

According to the sixth aspect of the present disclosure, it is possible to return an image quality evaluation that reflects actual usage by the user in the diagnosis of images outputted by an image processing device.

According to the seventh aspect of the present disclosure, it is possible to return an image quality evaluation that reflects actual usage by the user, as compared to a configuration that evaluates image quality on the basis of indiscriminate criteria.

According to the eighth aspect of the present disclosure, it is possible to return an image quality evaluation that reflects actual usage by the user on a computer in which the program is installed.

### Brief Description of the Drawings

An exemplary embodiment of the present disclosure will be described in detail based on the following figures, wherein:
Fig. 1 is a diagram illustrating a configuration of a diagnostic system to which an exemplary embodiment is applied;
Fig. 2 is a diagram illustrating a configuration of an image processing device;
Fig. 3 is a diagram illustrating an exemplary configuration of a computer that realizes a diagnostic server;
Fig. 4 is a diagram illustrating an exemplary configuration of a computer that realizes a terminal device;
Fig. 5 is a diagram illustrating an evaluation procedure by a diagnostic server;
Fig. 6 is a diagram illustrating an example of an application condition for the case where a diagnostic result is "NG";
Fig. 7 is a diagram illustrating an example of an application condition for the case where a diagnostic result is "Alert";
Fig. 8 is a diagram illustrating an example of an evaluation for which the diagnostic result is "NG"; and
Fig. 9 is a diagram illustrating an example of evaluation in the case where the evaluation item "staining" is excluded from evaluation in the example illustrated in Fig. 8.

### Detailed Description

Hereinafter, an exemplary embodiment of the present disclosure will be described in detail and with reference to the attached drawings.

### <System configuration>

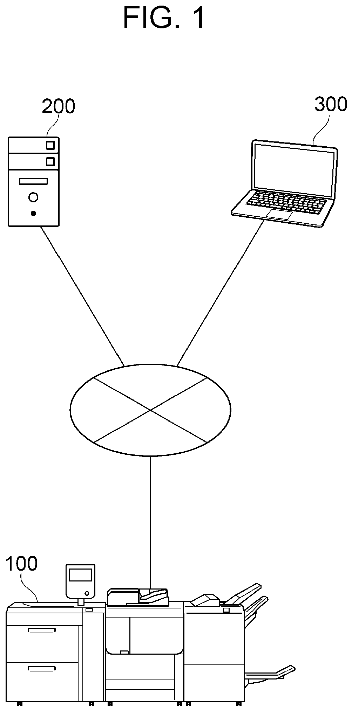

Fig. 1 is a diagram illustrating a configuration of a diagnostic system to which the exemplary embodiment is applied. The diagnostic system is provided with an image processing device 100, a diagnostic server 200, and a terminal device 300. The image processing device 100 is a device that forms and outputs an image using an image forming material such as toner on a recording material such as paper. The image processing device 100 also has a function to read and transmit an outputted image to the diagnostic server 200. The diagnostic server 200 is a server that evaluates an image received from the image processing device 100 and makes a diagnosis related to image quality. The terminal device 300 is an information processing device for obtaining a diagnostic result from the diagnostic server 200 and presenting the diagnostic result to an administrator or the like.

The image processing device 100 and the diagnostic server 200, and the diagnostic server 200 and the terminal device 300, are connected to one another over a network. The network is not particularly limited, and may be any network usable for data communication between devices. For example, a local area network (LAN), a wide area network (WAN), or the Internet may be used. The communication channel used for data communication may be wired, wireless, or a combination of the two. In one configuration, a relay device such as a gateway device, a router, or an access point may be used to connect devices through multiple networks and/or communication channels. The connection between the diagnostic server 200 and the terminal device 300 may use the same network as the network used for the connection between the image processing device 100 and the diagnostic server 200, or use a different network. The diagnostic server 200 and the terminal device 300 may also be connected directly in a case where the diagnostic server 200 is configured as a local server machine and placed in the same location as the terminal device 300.

### <Configuration of image processing device 100>

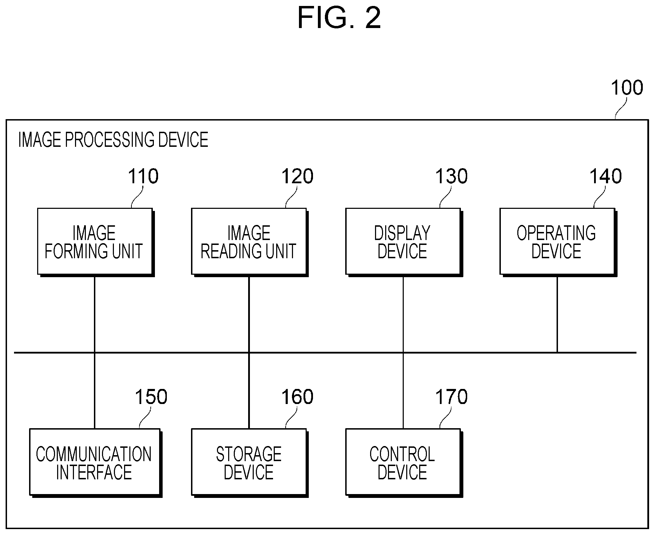

Fig. 2 is a block diagram illustrating a configuration of the image processing device 100. The image processing device 100 is provided with an image forming unit 110, an image reading unit 120, a display device 130, an operating device 140, a communication interface 150, a storage device 160, and a control device 170.

The image forming unit 110 uses an image forming material to form an image based on image data on a recording material. The method used to form an image on a recording material may be, for example, an electrophotographic method in which an image is formed by causing toner adhering to a photoconductor to be transferred to a recording material, or an inkjet method in which an image is formed by propelling ink onto a recording material.

The image reading unit 120 includes what is commonly called a scanner, and optically reads an image on a set document to generate data of a read image. The image reading method to be used may be, for example, a charge-coupled device (CCD) method in which light from a light source is emitted toward a document and the reflected light therefrom is focused by a lens and sensed by a CCD, or a contact image sensor (CIS) method in which light from light-emitting diode (LED) light sources is successively emitted toward a document and the reflected light therefrom is sensed by a CIS.

The display device 130 displays images, such as an informational image presenting various information to a user of the image processing device 100, a preview image of an image to be read, outputted, or otherwise processed, and an operating image enabling the user to perform operations. The display device 130 is a liquid crystal display, for example. The display device 130 and the operating device 140 may also be combined and used as a user interface by which the user inputs and outputs information with respect to the image processing device 100.

The operating device 140 enables the user to perform operations, such as entering commands and data. The operating device 140 includes, for example, hardware keys and a touch sensor that outputs a control signal according to a position pressed or touched by a finger or the like. The touch sensor and the liquid crystal display included in the display device 130 may also be combined to form a touch panel.

The communication interface 150 is an interface for transmitting and receiving commands and data to and from an external device. An interface suited to the method of communication with the external device is used as the communication interface 150. The connection with the external device may be a connection going through a network, or a direct connection. The communication channel may be a wired channel or a wireless channel. When the image processing device 100 includes a facsimile function, the communication interface 150 includes an interface for a telephone line.

The storage device 160 stores data and programs to be executed by the control device 170, the data of images read by the image reading unit 120 and the like, log data generated by various operations, and various other types of data. The storage device 160 is achieved with a storage device such as a magnetic disk drive or a solid-state drive (SSD), for example.

The control device 170 includes means for computation and storage, namely a processor and a memory, and performs various data processing and control of the image processing device 100 by loading a program stored in the storage device 160 into the memory and executing the program. For the processor, besides a central processing unit (CPU), a microprocessing unit (MPU), a graphics processing unit (GPU), a digital signal processor (DSP), or the like is used. For the memory, dynamic random access memory (DRAM) is used, for example.

### <Configuration of diagnostic server 200>

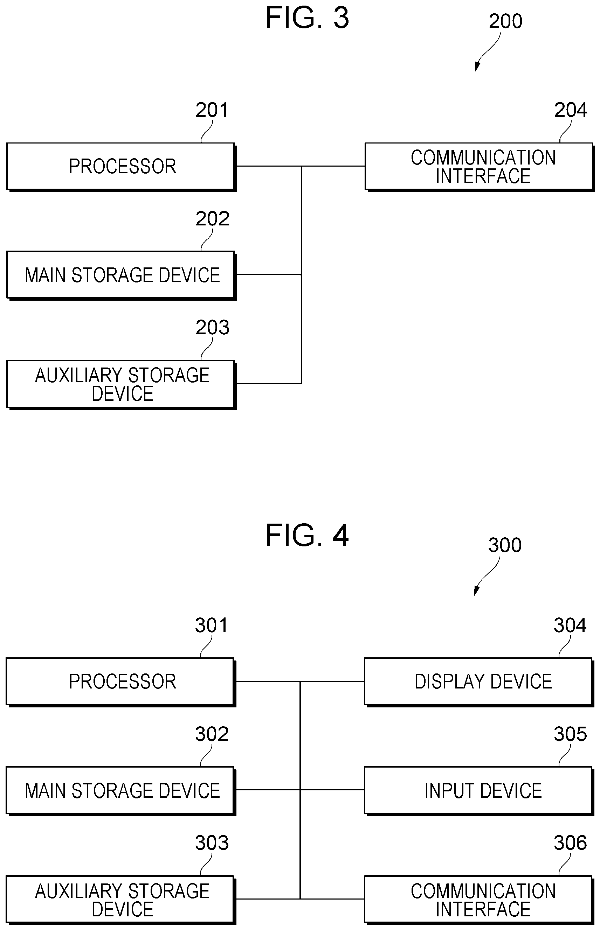

Fig. 3 is a diagram illustrating an exemplary configuration of a computer that realizes the diagnostic server 200. The diagnostic server 200 is provided with means for computation, namely one or more processors 201, and means for storage, namely a main storage device (main memory) 202 and an auxiliary storage device 203. The processor 201 achieves the functions of the diagnostic server 200 by loading a program stored in the auxiliary storage device 203 into the main storage device 202 and executing the program. For the processor 201, a central processing unit (CPU), microprocessing unit (MPU), graphics processing unit (GPU), or digital signal processor (DSP) is used, for example. For the main storage device 202, random access memory (RAM) is used, for example. For the auxiliary storage device 203, a magnetic disk drive or a solid-state drive (SSD) is used, for example. The diagnostic server 200 is also provided with a communication interface 204 for connecting to the image processing device 100 and the terminal device 300 over a network.

The diagnostic server 200 evaluates and diagnoses an image obtained from the image processing device 100 by having the processor 201 execute a program. Details regarding the evaluation and diagnosis of an image by the diagnostic server 200 will be described later. Note that the computer configuration illustrated in Fig. 3 is merely one example, and the computer realizing the diagnostic server 200 is not limited to the configuration illustrated in Fig. 3. For example, a configuration provided with non-volatile memory such as flash memory and read-only memory (ROM) as storage devices is also possible. Furthermore, the diagnostic server 200 is not limited to being a single computer as illustrated in Fig. 3, and may also be configured by distributing functions to multiple computers, or be realized as what is called a cloud server or the like built using resources on a network.

### <Configuration of terminal device 300>

Fig. 4 is a diagram illustrating an exemplary configuration of a computer that realizes the terminal device 300. The terminal device 300 is provided with means for computation, namely one or more processors 301, and means for storage, namely a main storage device (main memory) 302 and an auxiliary storage device 303. The processor 301 achieves the functions of the terminal device 300 by loading a program stored in the auxiliary storage device 303 into the main storage device 302 and executing the program. For the processor 301, a CPU, MPU, GPU, or DSP is used, for example. For the main storage device 302, RAM is used, for example. For the auxiliary storage device 303, a magnetic disk drive or SSD is used, for example.

The terminal device 300 is also provided with a display device 304 that displays various screens and an input device 305 that accepts input operations performed by a user. The display device 304 is a liquid crystal display, for example. The input device 305 includes a touch sensor and/or a keyboard, for example. The touch sensor of the input device 305 and the liquid crystal display of the display device 304 may also be combined to form a touch panel. The terminal device 300 is also provided with a communication interface 306 for connecting to the diagnostic server 200 over a network. For the terminal device 300, a smartphone, a tablet, or a laptop personal computer may be used, for example. Note that the computer configuration illustrated in Fig. 4 is merely one example, and the computer to be used as the terminal device 300 is not limited to the exemplary configuration in Fig. 4. For example, a configuration provided with non-volatile memory such as flash memory and ROM as storage devices is also possible.

### <Evaluation and diagnosis of image by diagnostic server 200>

Next, the evaluation and diagnosis of an image by the diagnostic server 200 will be described. As preliminary operations, an image formed on a recording material by the image forming unit 110 of the image processing device 100 is outputted, and the outputted image is read by the image reading unit 120 to obtain read data (image data) of the output image. Thereafter, the image data is transmitted from the image processing device 100 to the diagnostic server 200 and subjected to evaluation and diagnosis by the diagnostic server 200.

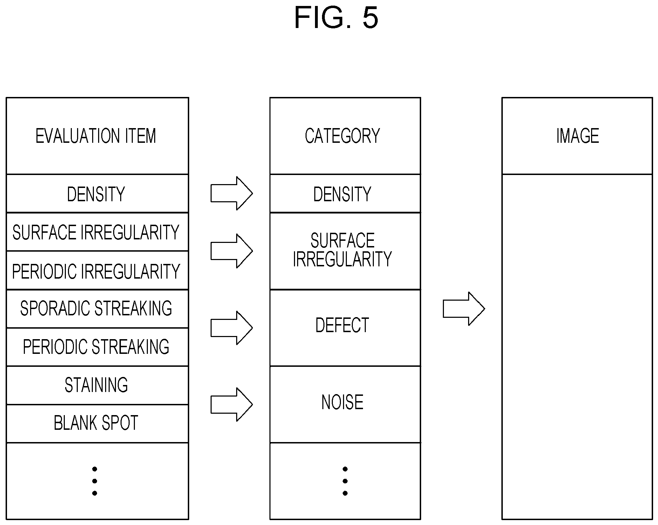

Fig. 5 is a diagram illustrating an evaluation procedure by the diagnostic server 200. In the evaluation of an image by the diagnostic server 200, first, an evaluation is made with respect to evaluation items pertaining to image quality divided into multiple categories. An evaluation for each category is made on the basis of the evaluation results for each of the evaluation items, and an evaluation of the image quality of the overall image is made on the basis of the evaluation results for each of the categories. The evaluations for each of the evaluation items are given by points (hereinafter referred to as "evaluation points") specified according to a preset evaluation criterion for each evaluation item. In the example illustrated in Fig. 5, "density", "surface irregularity", "periodic irregularity", "sporadic streaking", "periodic streaking", "staining", "blank spot", and the like are set as evaluation items, and "density", "surface irregularity", "defect", "noise", and the like are set as categories. The evaluation item "density" is included in the category "density". The evaluation item "surface irregularity" and the evaluation item "periodic irregularity" are included in the category "surface irregularity". The evaluation items "sporadic streaking" and "periodic streaking" are included in the category "defect". The evaluation items "staining" and "blank spot" are included in the category "noise".

As above, the diagnostic server 200 first specifies evaluation points for each evaluation item with respect to an image to be diagnosed, on the basis of an evaluation criterion for each evaluation item. The evaluation items and evaluation criteria are not particularly limited. The specific settings of the evaluation items and evaluation criteria may be set by a provider of the diagnostic system or by a user of the diagnostic system. The user may also be able to modify initial settings set by the provider of the diagnostic system. The category classifications similarly may be configured to be set by the provider or user of the diagnostic system. The diagnostic server 200 calculates evaluation points for each category on the basis of the evaluation points for each of the evaluation items specified on the basis of the evaluation criteria, and further calculates, on the basis of the evaluation points for each of the categories, evaluation points for the image quality of the overall image to be diagnosed.

As an example, the following describes a method of calculating evaluation points for each category by weighting the evaluation points for each evaluation item in the same category. For instance, suppose that the maximum number of evaluation points is 100 points and the evaluation points for the evaluation items "surface irregularity" and "periodic irregularity" included in the category "surface irregularity" illustrated in Fig. 5 are A1 points and A2 points, respectively. In addition, suppose that the evaluation points A1 and A2 have a relationship in which A1 < A2. The values (100 - A1) and (100 - A2) obtained by subtracting the evaluation points for each evaluation item from the maximum number of 100 points are then multiplied by predetermined weight values in order from the highest numerical value. For example, suppose that x1 is the weight value with respect to the highest numerical value and x2 is the weight value with respect to the second-highest numerical value. Since A1 < A2, it follows that (100 - A1) > (100 - A2). Consequently, the results of multiplying by the weight values are (100 - A1) × x1 for the calculated value of the evaluation item "surface irregularity", and (100 - A2) × x2 for the calculated value of the evaluation item "periodic irregularity". The value obtained by subtracting these calculated values from the maximum number of 100 is then obtained as the points for the category. In other words, in the above case, the points for the category "surface irregularity" is calculated according to 100 - (100 - A1) × x1 - (100 - A2) × x2.

Evaluation points reflecting weightings corresponding to the evaluation points for each of the evaluation items for other categories are calculated in a similar manner. Evaluation points for the overall image are then calculated on the basis of the evaluation points for each of the categories and the weightings corresponding to the evaluation points. Note that the above calculation method is merely one example of a method of specifying evaluation points for categories on the basis of evaluation points for evaluation items, and the method of specifying evaluation points for categories is not limited to the above method. Moreover, in the above calculation method, too, the specific weight values and the number of evaluation items to be used to calculate the evaluation points for a category may be set individually by the provider of the diagnostic system or the like.

Next, image diagnosis based on evaluation points will be described. Herein, as an example, the state of an image is determined in the three stages of "OK", "Alert", and "NG" on the basis of the evaluation points for individual evaluation items. "OK" is a diagnostic result indicating that no issues have occurred in any of the evaluation items. "Alert" is a diagnostic result indicating that a mild issue has occurred in one or more evaluation items. "NG" is a diagnostic result indicating that a sever issue has occurred in one or more evaluation items.

The relationship between the evaluation points for evaluation items and the diagnostic result of an image is set as follows, for example.
"OK": evaluation points ≥ 80 points
"Alert": 80 points > evaluation points ≥ 20 points
"NG": 20 points > evaluation points
In this case, if the evaluation points for any evaluation item are less than 20 points, the diagnostic result of the image is "NG". If the evaluation points for all evaluation items are 20 points or more but the evaluation points for any evaluation item is less than 80 points, the diagnostic result of the image is "Alert". If the evaluation points for all evaluation items are 80 points or more, the diagnostic result of the image is "OK". When the diagnostic result is "Alert" or "NG", the user may identify the evaluation item that is the cause of such a diagnostic result and take measures for improving image quality with respect to the image processing device 100, such as changing the print settings.

When the above diagnostic result meets a predetermined application condition, the diagnostic server 200 prompts the user to determine whether or not to change the evaluation method regarding image quality. The application condition may be set on the basis of evaluation results (diagnostic results) in multiple consecutive evaluations, for example. Specific examples of the application condition will be described later.

The specific changes to the evaluation method are different depending on whether the diagnostic result is "Alert" or "NG". For example, when the output of an image with a diagnostic result of "NG" meets the application condition, an operation may be performed such that the evaluation item that is the cause of such a diagnostic result is excluded from evaluation, and when the output of an image with a diagnostic result of "Alert" meets the application condition, an operation may be performed such that the evaluation criterion for the evaluation item that is the cause of such a diagnostic result is relaxed.

When the diagnostic result meets the application condition, the diagnostic server 200 transmits, to the terminal device 300, a message querying the user about whether or not implement a change of the evaluation method in the case where the diagnostic result is "Alert" or "NG". The user uses the terminal device 300 to refer to the message obtained from the diagnostic server 200, decides whether or not to implement a change of the evaluation method, and responds to the diagnostic server 200.

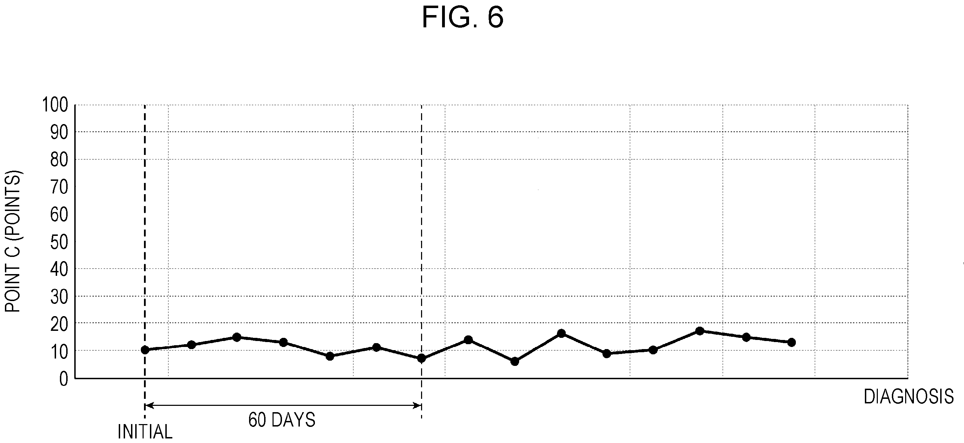

Fig. 6 is a diagram illustrating an example of the application condition for the case where the diagnostic result is "NG". In the example illustrated in Fig. 6, image evaluation and diagnosis is performed every 10 days, and image output in which the evaluation points are less than 20 points (and consequently, the diagnostic result is "NG") occurs consecutively in relation to a certain evaluation item ("Point C" in the diagram). In this example, "NG" has continued for 60 days from the occurrence of the initial "NG" (the period indicated by the dashed lines in the diagram), and the diagnostic result has not improved. In other words, "NG" has occurred because "Point C" has been less than 20 points seven times consecutively. In such a case, the diagnostic server 200 transmits a message prompting for a determination on a change of the evaluation method to the terminal device 300. For example, the content of the message is assumed to be like the following: "Evaluation item 'Point C' has not improved for 60 days. Exclude this item from the calculation of evaluation points?" The user refers to the message displayed on the terminal device 300, determines whether or not to exclude the evaluation item "Point C" from the calculation of evaluation points, and responds from the terminal device 300 to the diagnostic server 200.

For example, in some situations, the user may not consider a certain evaluation item ("Point C" in this case) in relation to images that the user outputs, and may not regard it as an issue even if the element of image quality corresponding to the evaluation item is degraded, in which case the user will not change settings or the like to improve image quality in the image processing device 100. As a result, as illustrated in Fig. 6, the image evaluation will continue to be in a state with low evaluation points for the relevant evaluation item. Accordingly, the diagnostic server 200 prompts for a determination prompts for a determination on whether or not to exclude the relevant evaluation item from evaluation.

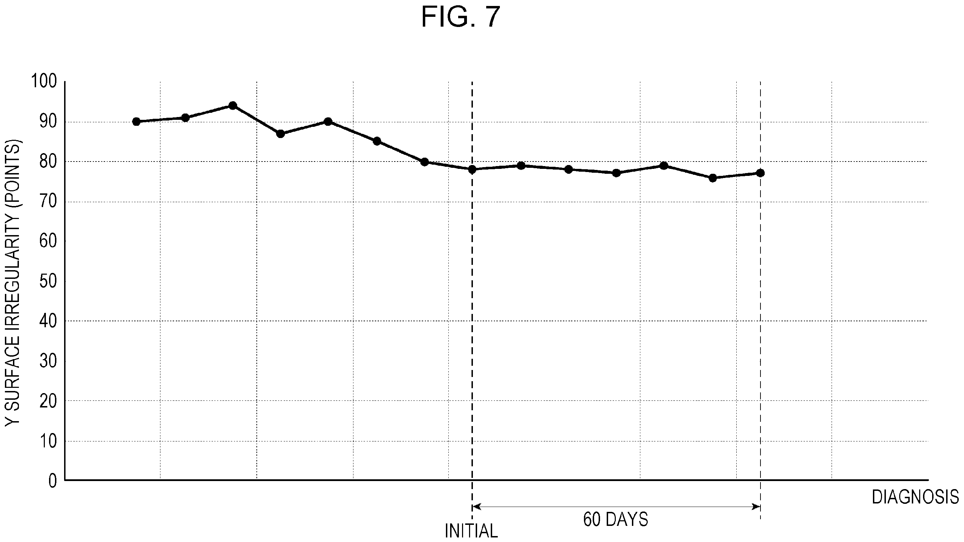

Fig. 7 is a diagram illustrating an example of the application condition for the case where the diagnostic result is "Alert". In the example illustrated in Fig. 7, image evaluation and diagnosis is performed every 10 days, and image output in which the evaluation points are less than 80 points (and consequently, the diagnostic result is "Alert") occurs consecutively in relation to a certain evaluation item ("Y surface irregularity" in the diagram). In this example, "Alert" has continued for 60 days from the occurrence of the initial "Alert" (the period indicated by the dashed lines in the diagram), and the diagnostic result has not improved. In other words, "Alert" has occurred because "Y surface irregularity" has been less than 80 points seven times consecutively. In such a case, the diagnostic server 200 transmits a message prompting for a determination on a change of the evaluation method to the terminal device 300. For example, the content of the message is assumed to be like the following: "Evaluation item 'Y surface irregularity' has not improved for 60 days. Please check the image quality related to this item. If there is no issue with the image quality, would you like to change the evaluation criterion so that the image quality level of the current output is OK?" The user refers to the message displayed on the terminal device 300, determines whether or not to relax the evaluation criterion for the evaluation item "Y surface irregularity", and responds from the terminal device 300 to the diagnostic server 200.

The foregoing describes image evaluation and diagnosis by the diagnostic server 200, but the setting of evaluation items and categories in the evaluation of images described above, the calculation method and the setting of weight values of the evaluation points for categories based on the evaluation points for evaluation items, the calculation method and the setting of weight values of the evaluation points for images based on the evaluation points for categories, the types of diagnostic results, the threshold values for the evaluation points for images associated with each diagnostic result, and the like are merely illustrative examples. Any of various existing techniques may be applied to the present exemplary embodiment insofar as the technique is used to evaluate an image to be diagnosed, specify evaluation items related to image quality, and diagnose an image on the basis of an evaluation of each evaluation item.

### <Calculation examples>

Next, specific examples of calculating evaluation points will be presented to describe how a change in the evaluation method influences the evaluation points and the diagnostic result. As an example, the following illustrates the influence on the evaluation points and the diagnostic result due to a change in the evaluation method when the diagnostic result is "NG".

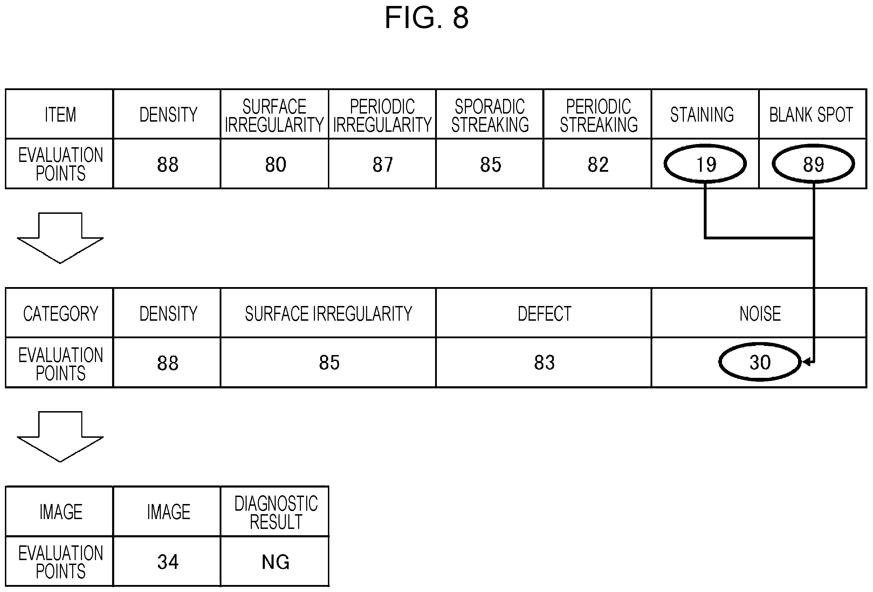

Fig. 8 is a diagram illustrating an example of an evaluation for which the diagnostic result is "NG". In the example illustrated in Fig. 8, "density": 88 points, "surface irregularity": 80 points, "periodic irregularity": 87 points, "sporadic streaking": 85 points, "periodic streaking": 82 points, "staining": 19 points, and "blank spot": 89 points are indicated as the evaluation points for each of the evaluation items. Also, "density": 88 points, "surface irregularity": 85 points, "defect": 83 points, and "noise": 30 points are indicated as the evaluation points for each of the categories calculated on the basis of the evaluation points for each of the evaluation items. In addition, 34 points is indicated as the evaluation points for the image quality of the image overall, which is calculated on the basis of the evaluation points for each of the categories. The diagnostic result for the image is "NG". Note that the setting of the weight values used in the calculations of the evaluation points is not specified herein, and only the numerical values of the calculation results are indicated.

In the example illustrated in Fig. 8, the evaluation points for the evaluation item "staining" are low, and because of this, the evaluation points for the category "noise" and the evaluation points for the image overall are low values. In addition, the diagnostic result for the image is "NG". In some cases, the user of the image processing device 100 may not scrutinize the presence or absence of staining in relation to images outputted using the image processing device 100, and may tolerate staining to the degree of the evaluation points indicated in Fig. 8. In this case, it is conceivable that even though the diagnostic results is "NG", the user may continue to use the image processing device 100 without changing settings for improving the image quality related to the evaluation item "staining". Accordingly, the evaluation item "staining" is excluded from evaluation.

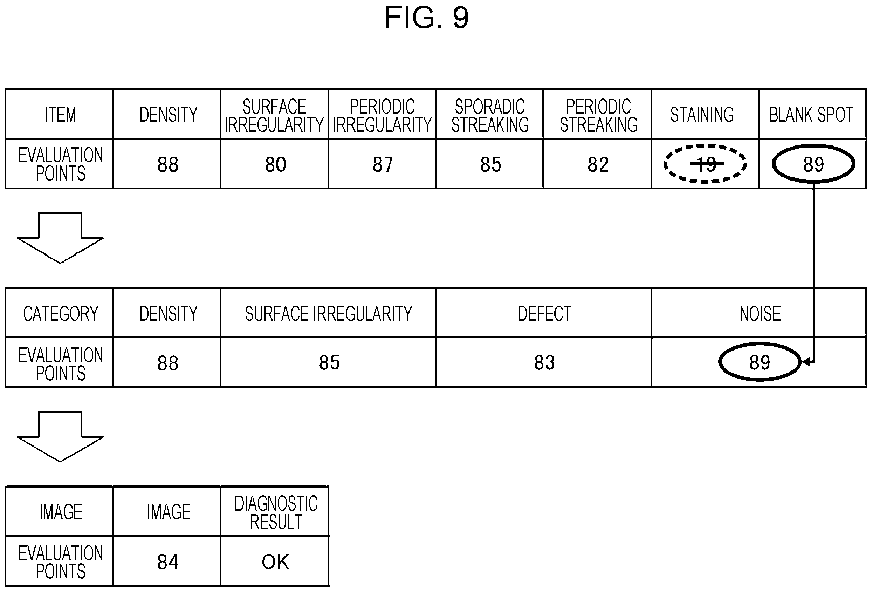

Fig. 9 is a diagram illustrating an example of evaluation in the case where the evaluation item "staining" is excluded from evaluation in the example illustrated in Fig. 8. In the example illustrated in Fig. 9, the evaluation points for the evaluation item "staining" have been removed (expressed in the diagram by a strikethrough line applied to the numerical value). For this reason, only the 89 points which are the evaluation points for the evaluation item "blank spot" are reflected in the evaluation points for the category "noise". Additionally, with this change in the evaluation points for the category "noise", the evaluation points for the image quality of the image overall is 84 points, and the diagnostic result for the image is "OK". Consequently, the evaluation and diagnostic result by the diagnostic server 200 with respect to an image outputted by the image processing device 100 are more in line with what the user of the image processing device 100 expects, as compared to the example illustrated in Fig. 7.

The foregoing describes an exemplary embodiment of the present disclosure, but the technical scope of the present disclosure is not limited to the foregoing exemplary embodiment. For example, in the exemplary embodiment above, the application condition for prompting the user to determine whether or not to change the evaluation method regarding image quality is set on the basis of the evaluation results (diagnostic results) from multiple consecutive evaluations. Additionally, the same diagnostic result continuing for 60 days (or seven times consecutively) from the occurrence of the initial diagnostic result ("NG" or "Alert") is presented as a specific application condition. The period of continuation (continual number of times) is merely an illustrative example and may be another period or number of times. The application conditions may also be set on the basis of the percentage of specific evaluation results or diagnostic results, the trend in evaluation results, or the like among the diagnostic results in a certain period or a certain number of diagnostic results. In the case where an "NG" or "Alert" diagnostic result is returned, after which the diagnostic result becomes "OK" due to a setting change in the image processing device 100, and then "NG" or "Alert" occurs again, there is a possibility that the cause is different from that of the diagnostic result from before the setting change. Consequently, in such a case, the diagnostic server 200 may also be configured not to transmit a message prompting the user to determine whether or not to change the evaluation method regarding image quality.

Also, in the exemplary embodiment above, an image formed on a recording material by the image forming unit 110 of the image processing device 100 is read by the image reading unit 120 of the same image processing device 100 to obtain the image data to be diagnosed. In contrast, an image outputted from the image processing device 100 may be read by another image reading device, and the obtained image data may be provided to the diagnostic server 200 as image data to be diagnosed. Otherwise, various modifications and substitutions that do not depart from the scope of the technical ideas of the present disclosure are also included in the present disclosure.

In the embodiments above, the term "processor" refers to hardware in a broad sense. Examples of the processor include general processors (e.g., CPU: Central Processing Unit) and dedicated processors (e.g., GPU: Graphics Processing Unit, ASIC: Application Specific Integrated Circuit, FPGA: Field Programmable Gate Array, and programmable logic device).

In the embodiments above, the term "processor" is broad enough to encompass one processor or plural processors in collaboration which are located physically apart from each other but may work cooperatively. The order of operations of the processor is not limited to one described in the embodiments above, and may be changed.

The foregoing description of the exemplary embodiments of the present disclosure has been provided for the purposes of illustration and description. It is not intended to be exhaustive or to limit the disclosure to the precise forms disclosed. Obviously, many modifications and variations will be apparent to practitioners skilled in the art. The embodiments were chosen and described in order to best explain the principles of the disclosure and its practical applications, thereby enabling others skilled in the art to understand the disclosure for various embodiments and with the various modifications as are suited to the particular use contemplated. It is intended that the scope of the disclosure be defined by the following claims and their equivalents.

### <Appendix>

(((1))) An information processing system comprising:
   at least one processor configured to:
   obtain image data to be diagnosed;
   evaluate the image data on the basis of evaluation criteria for each of one or more evaluation items related to image quality; and
   allow an evaluation method based on the evaluation criteria to be changed according to an evaluation result in the evaluation of each of the evaluation items.
(((2))) The information processing system according to (((1))), wherein the processor is configured such that when the evaluation result meets a predetermined condition in relation to one evaluation item, the processor allows the evaluation item to be excluded from evaluation.
(((3))) The information processing system according to (((2))), wherein the processor is configured such that when the evaluation result meets a second condition different from the predetermined condition in relation to one evaluation item, the processor allows the evaluation criterion for the evaluation item to be relaxed.
(((4))) The information processing system according to any one of (((1))) to (((3))), wherein the processor is configured such that when evaluation results from multiple consecutive evaluations meet a predetermined condition in relation to one evaluation item, the processor allows the evaluation method regarding the evaluation item to be changed.
(((5))) The information processing system according to (((4))), wherein the processor is configured such that when evaluation results from multiple evaluations meet a predetermined condition in relation to one evaluation item in an ongoing way, the processor allows the evaluation method regarding the evaluation item to be changed.
(((6))) A diagnostic system comprising:
   the information processing system according to any one of (((1))) to (((5)));
   an image processing device that forms an image on a recording medium; and
   a reading device that reads an image formed on a recording medium by the image processing device and creates image data to be diagnosed.
(((7))) A program causing a computer to execute a process comprising:
   obtaining image data to be diagnosed;
   evaluating the image data on the basis of evaluation criteria for each of one or more evaluation items related to image quality; and
   allowing an evaluation method based on the evaluation criteria to be changed according to an evaluation result in the evaluation of each of the evaluation items.

According to the information processing system as in (((1))), it is possible to return an image quality evaluation that reflects actual usage by the user, as compared to a configuration that evaluates image quality on the basis of indiscriminate criteria.

According to the information processing system as in (((2))), an evaluation item that the user deems unnecessary may be excluded from evaluation.

According to the information processing system as in (((3))), the evaluation criterion for an evaluation item that the user does not scrutinize may be relaxed.

According to the information processing system as in (((4))), user preferences with respect to image quality may be reflected, unlike a configuration that makes a determination on the basis of a single evaluation result.

According to the information processing system as in (((5))), it is possible to exclude cases in which the evaluation results vary irregularly due to a fault or the like.

According to the diagnostic system as in (((6))), it is possible to return an image quality evaluation that reflects actual usage by the user in the diagnosis of images outputted by an image processing device.

According to the program as in (((7))), it is possible to return an image quality evaluation that reflects actual usage by the user on a computer in which the program is installed.

## Claims

1. An information processing system comprising:
at least one processor configured to:
obtain image data to be diagnosed;
evaluate the image data on the basis of evaluation criteria for each of one or more evaluation items related to image quality; and
allow an evaluation method based on the evaluation criteria to be changed according to an evaluation result in the evaluation of each of the evaluation items.

2. The information processing system according to claim 1, wherein the processor is configured such that when the evaluation result meets a predetermined condition in relation to one evaluation item, the processor allows the evaluation item to be excluded from evaluation.

3. The information processing system according to claim 2, wherein the processor is configured such that when the evaluation result meets a second condition different from the predetermined condition in relation to one evaluation item, the processor allows the evaluation criterion for the evaluation item to be relaxed.

4. The information processing system according to any one of claims 1 to 3, wherein the processor is configured such that when evaluation results from multiple consecutive evaluations meet a predetermined condition in relation to one evaluation item, the processor allows the evaluation method regarding the evaluation item to be changed.

5. The information processing system according to claim 4 , wherein the processor is configured such that when evaluation results from multiple evaluations meet a predetermined condition in relation to one evaluation item in an ongoing way, the processor allows the evaluation method regarding the evaluation item to be changed.

6. A diagnostic system comprising:
the information processing system according to any one of claims 1 to 5;
an image processing device that forms an image on a recording medium; and
a reading device that reads an image formed on a recording medium by the image processing device and creates image data to be diagnosed.

7. An information processing method comprising:
obtaining image data to be diagnosed;
evaluating the image data on the basis of evaluation criteria for each of one or more evaluation items related to image quality; and
allowing an evaluation method based on the evaluation criteria to be changed according to an evaluation result in the evaluation of each of the evaluation items.

8. A program causing a computer to execute a process comprising:
obtaining image data to be diagnosed;
evaluating the image data on the basis of evaluation criteria for each of one or more evaluation items related to image quality; and
allowing an evaluation method based on the evaluation criteria to be changed according to an evaluation result in the evaluation of each of the evaluation items.
